# EUROPEAN PATENT SPECIFICATION

(11) **EP 1 700 117 B1**
(45) Date of publication and mention of the grant of the patent: **21.05.2008**
(21) Application number: 03819198.7
(22) Date of filing: 31.12.2003
(51) Int. Cl.: G01N 33/50, C12Q 1/00

(54) **METHOD AND KIT FOR PESTICIDE ANALYSIS**
VERFAHREN UND VORRICHTUNG ZUR ANALYSE VON PESTIZIDEN
PROCEDE ET KIT D'ANALYSE DE PESTICIDES

(43) Date of publication of application: 13.09.2006
(73) Proprietor: COUNCIL OF SCIENTIFIC AND INDUSTRIAL RESEARCH, New Delhi 110 001 (IN)
(72) Inventor: THAKUR, Munna, Singh, Mysore 570 013 (IN); KARANTH, Naikankatte, Ganesh, 570 013 Karnataka (IN); KUMAR, Mysore, Anantharamaiah, 570 013 Karnataka (IN); AMITA RANI, Bangalore, Eshwar, 570 013 Karnataka (IN); PASHA, Akimal, 570 013 Karnataka (IN); KARANTH, Nittiru, Gopala, Krishna, 570 013 Karnataka (IN)
(74) Representative: Viering, Jentschura & Partner
(86) International application number: PCT/IN2003/000446
(87) International publication number: WO 2005/064331

(56) References cited:
- US-A- 5 900 944
- REKHA K ET AL: "BIOSENSORS FOR THE DETECTION OF ORGANO-PHOSPHOROUS PESTICIDES" CRC CRITICAL REVIEWS IN BIOTECHNOLOGY, CRC PRESS, BOCA RATON, FL, US, vol. 20, no. 3, 2000, pages 213-235, XP001028933 ISSN: 0738-8551
- REKHA K ET AL: "Ascorbate oxidase based amperometric biosensor for organophosphorous pesticide monitoring" BIOSENSORS AND BIOELECTRONICS, vol. 15, no. 9-10, November 2000 (2000-11), pages 499-502, XP002285566 ISSN: 0956-5663
- GOUDA M D ET AL: "A DUAL ENZYME AMPEROMETRIC BIOSENSOR FOR MONITORING ORGANOPHOSPHOROUS PESTICIDES" BIOTECHNOLOGY TECHNIQUES, CHAPMAN & HALL, XX, vol. 11, no. 9, September 1997 (1997-09), pages 653-655, XP001028921 ISSN: 0951-208X
- LEHOTAY S J ET AL: "Application of gas chromatography in food analysis" TRAC, TRENDS IN ANALYTICAL CHEMISTRY, ANALYTICAL CHEMISTRY. CAMBRIDGE, GB, vol. 21, no. 9-10, 10 September 2002 (2002-09-10), pages 686-697, XP004387123 ISSN: 0165-9936
- ANASTASSIADES M ET AL: "Evaluation of analyte protectants to improve gas chromatographic analysis of pesticides" JOURNAL OF CHROMATOGRAPHY A, ELSEVIER SCIENCE, NL, vol. 1015, no. 1-2, 10 October 2003 (2003-10-10), pages 163-184, XP004458238 ISSN: 0021-9673
- KOCHMAN M ET AL: "Fast, high-sensitivity, multipesticide analysis of complex mixtures with supersonic gas chromatography-mass spectrometry" JOURNAL OF CHROMATOGRAPHY A, ELSEVIER SCIENCE, NL, vol. 974, no. 1-2, 18 October 2002 (2002-10-18), pages 185-212, XP004387549 ISSN: 0021-9673
- SKERRITT J H ET AL: "Sensitive immunoassays for methyl-parathion and parathion and their application to residues in foodstuffs." FOOD AND AGRICULTURAL IMMUNOLOGY, vol. 15, no. 1, March 2003 (2003-03), pages 1-15, XP009032616 ISSN: 0954-0105 (ISSN print)

## Description

### Field of the invention

The present invention provides a rapid test kit for pesticide analysis based on charge coupled device. The present invention also provides a method for the manufacture of a charge coupled device based test kit for pesticide analysis and to the use thereof for determination of pesticide levels in water, food and environment.

### Background of the invention

Environmental protection in connection with water and agriculture need urgent attention for human health. Major health problems occur due to the use of contaminated water and food. Application of organochlorine and organophosphosporous pesticides in agriculture has been practiced in many countries. As a result of increased use of pesticides in agriculture in the last few decades, ground water, raw food materials and processed food are becoming contaminated with pesticide residues.

The pollution monitoring and protection agencies need rapid and sensitive methods for the analysis of pollutants. The commonly used analytical methods for pesticide analysis include Liquid chromatography, Gas chromatography and ELISA methods (Lehotay, S.J., analysis of pesticide residues in mixed fruit and vegetable extracts by direct sample introduction/ Gas chromatography/tandem mass spectrometry, J. AOAC Int., 2002, 83, 680-697; Sasaki. K., Suzuki.K., and Sito. Y., (1987) 'A simplified cleanup and gas chromatographic determination of organophosphorous pesticides in crops' Journal of the Association of Official analytical Chemists International, 70, 460-464. Fernandez. M., Pico, Y., Girotti, S., Manes, J., 'Analysis of organophosphorous pesticides in honey bee by liquid chromatography-atmospheric pressure chemical ionization- mass spectrometry', J. Agric. Food chem. 2001, 49, 3540-3547).

These conventional methods are sensitive upto ppb level, but are time consuming, laborious and require skilled technicians and expensive instrumentation. Biosensor is an alternative instrument method to detect the pesticide rapidly and economically. Literature on the applications of biosensor for rapid qualitative detection of pesticide is scanty. In particular, the qualitative rapid techniques are not available to detect methyl parathion (MP) pesticide residues in water and food materials for field application.

Reference is made to US Patent 6,569,384 (Inventors: Greenbaum; Elias (Oak Ridge, TN); Sanders; Charlene A. (Knoxville, TN) May 27, 2003, 'Tissue-based water quality biosensors for detecting chemical warfare agents'). They have described an apparatus for introducing water into the cell and discharging water from the cell adapted for analyzing photosynthetic activity of naturally occurring, free-living, indigenous photosynthetic organisms in water; a fluorometer for measuring photosynthetic activity of these organisms drawn into the cell; and an electronics package that analyzes raw data from the fluorometer and emits a signal indicating the presence of at least one pesticide at µg/ml (ppm level). Limitation of this method is detection of the pesticide up to ppb level only and also time consuming and not specific to any pesticides. This method will respond nonspecifically to many organophosphorous and carbamate pesticides.

Reference is made to a dipstick immunoassay method described by Mosiello, L., Cremisini, C., Sergre, L., Chiavarini, S. and Spano, M. (1998) Dipstick Immunoassay format for atrazine and Terbuthylazine analysis in water samples. J. Agric. Food Chem. 46, 3847-3851. In this method antibodies were immobilised on nylon membrane and detection limit was 1.2 to 10µg/L⁻¹ (ppb level) when they use reflectometer. Detection limit of this method is ppb level. As has been reported, the main problem existing in this method is the poor precision, probably due to problems in the homogeneity of the monoclonal antibody on the nylon membrane or polystyrol transperants and the instability of the coloured TMB charge-transfer complex.

Reference may be made to a Test Kit and method for the determination of pesticides by Eliezer, Z, Steven, S. Stanely E.C, Sciences, C in W093 14222. In this publication, the use of insect brain as the biological sensing agent has been described. The publication, however, does not describe the detection limit for the pesticide or the duration required for the analysis.

The prior art methods reported above are time consuming. In addition, most of the known methods are not specific to the analyte and a sound technical knowledge is required for the user. Therefore, there is a need for an improved method and test kit for use in pesticide analysis.

### Objects of the invention

It is therefore, an important object of the present invention to provide a rapid test kit for pesticide detection, which obviates the above drawbacks.

It is another object of the present invention to provide a rapid test kit for pesticide detection at high sensitivity (ppt Level) using the immuno-chemiluminescence principle.

### Summary of the invention

The above and other objects of the present invention are achieved by the present invention by utilizing in a novel manner, chemiluminescence (CL) and biochemical reactions to produce light, which is proportional to pesticides concentration at high sensitivity (ppt level). There is no information available at present on determination of pesticide specially Mehyl /Ethyl Parathion (MP/EP) using chemiluminescence (CL). It is known that CL based test kit is very sensitive to detect the analyte very low concentrations. CL method has numerous advantages such as sensitive rapid assay and possibility of robust and inexpensive instrumentation and hence this test kit has become an attractive analytical tool in pesticide determination. In this connection, reference is made to (J Wang C. Zhang, H. Wang, F. Yang and X. Zhang, 2001, Development of a luminol based chemilumnescence flow injection method for the determination of dichlorvos pesticide, 54, 1185-1193) in which they have detected diclorvos at ppm level (0.2 - 3.1µg/ml). However, this method does not work for detection of methyl parathion. It should be noted that there was no involvement of antibody-antigen reactions in the prior art methods mentioned above. On the contrary, the novelty of the present invention resides in the use of antibody-antigen reactions, which are specific to pesticides coupled with a charge-coupled device (CCD). There are no existing reports to detect pesticides at high sensitivities (upto ppt level) using chemiluminescence and CCD camera. The CL based test kit developed in the present invention enables rapid and sensitive detection of pesticide upto ppt level. Another novelty of the present invention resides in the use of an electron mediator such as potassium ferricayanide, to amplify the very low level of light signals produced, thereby enabling clear distinction of different pesticide concentrations from ppm to ppt levels.

### Detailed Description

The present invention will now be described in detail with reference to the accompanying drawings wherein:
Fig 1 shows the HRP enzyme catalysed oxidation of electron donor substrate with hydrogen peroxide; and
Fig 2 shows a flow chart of the use of the pesticide test kit in accordance with the present invention.

### Principle of Immuno-chemiluminescence (ICL) based pesticide detection:

The present invention is based on the finding that in the presence of hydrogen peroxide (H₂O₂), horseradish peroxidase (HRP) catalyzes the oxidation of luminol. Immediately following the oxidation of luminol, it reaches an excited state, which then decays to the ground state by emitting light. Using this method it is possible to detect pesticides such as ethyl parathion (EP) and Methyl parathion, using respective antibodies.

HRP enzyme catalyzes oxidation of electronic donor substrates with hydrogen peroxide according to the following scheme.

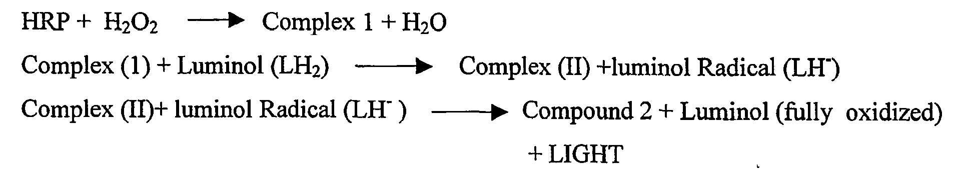

### Pesticide Detection:

MP antibodies are immobilised in excess on the carrier matrix in the immunobioreactor column. Pesticide molecules bind with the immobilized antibodies. During this process some of the antibody sites (epitopes) remain unbound. These unbound sites are available for binding the MP-HRP-conjugate. A fixed amount of MP-HRP-conjugate was passed through the column, thereby the remaining antibody epitope sites are occupied by this conjugate. This antibody column was now washed thoroughly. These immobilised matrix-having pesticide and MP-HRP-Conjugate was taken out and subjected to chemiluminescence reaction as given above. However, in this reaction light produced is of very low intensity. To get enhanced light intensity the electron mediator K₃FeCN₆ is added. At high pesticide concentration having more unreacted H₂O₂, K₃FeCN₆ reacts with this and produces an enhanced light intensity which is directly proportional to the pesticide concentration.

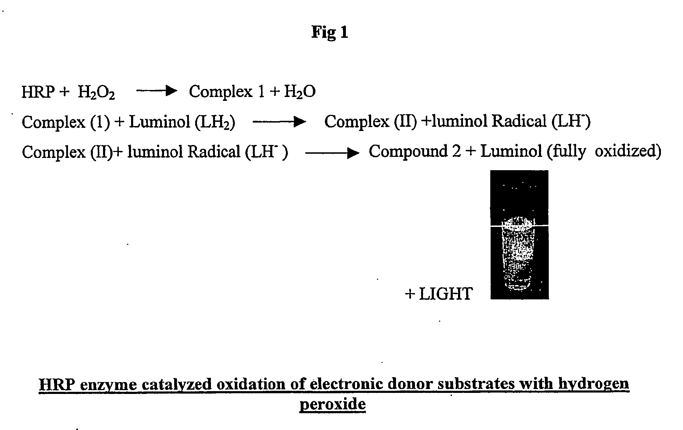

As shown in Fig 1, in the presence of hydrogen peroxide (H₂O₂), horseradish peroxidase (HRP) catalyzes the oxidation of luminol. Immediately following the oxidation of luminol, it reaches an excited state, which then decays to the ground state by emitting light. Using this method it is possible to detect pesticide such as ethyl parathion (EP) and Methyl parathion, using respective antibodies.

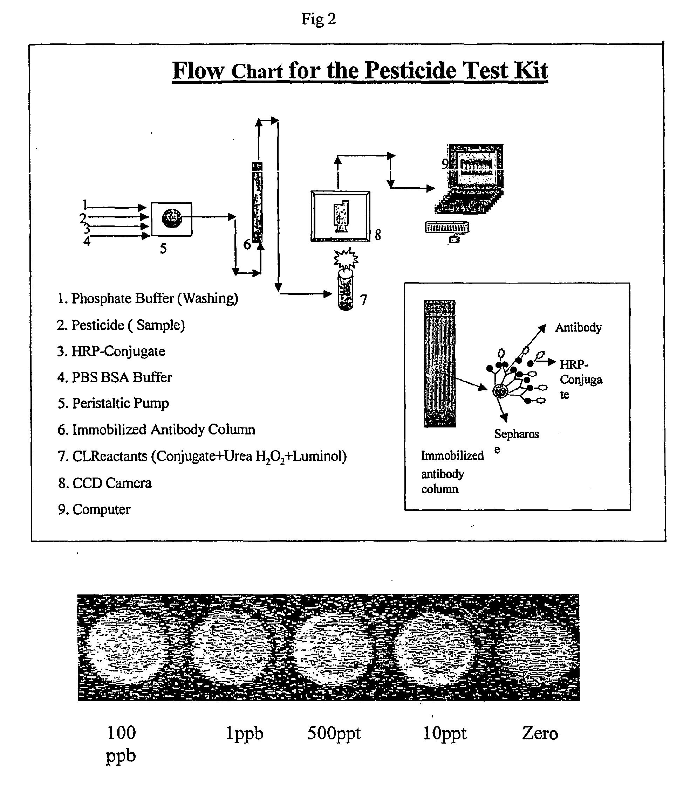

Fig.2 illustrates the method and the kit (or device) in accordance with the present invention. A kit (or device) in accordance with the present invention for rapid detection of pesticide at ppt level using immuno-chemiluminescence comprises a capillary column 6 for packing immobilized antibodies. A source of sample solution 2 to be tested for pesticide content is operatively connected to said column 6. A source of buffer 1 is operatively connected to said column for equilibrating the antibody and washing unbound pesticides. Sources of urea H₂O₂ Luminol and K₃Fe(CN)₆ also operatively connected to said column 6. Preferably, all said sources of reagents are connected to said column 6 through a peristaltic pump 5. The mixing said reagents results in light being generated, which may take place I column 7 connected to said column 6. The light generated is captured by a CCD camera 8 to record the intensity of the light produced which is linked to a computer 9 for effecting comparison with a control sample.

A preferred embodiment of the method of detecting the presence of pesticide in water, in accordance with the present invention comprises the following:
(1). Methyl parathion (MP) antibodies raised in chickens and purified from egg yolk (IgY- MP) and immobilized on sepharose matrix through reductive amination (Hermanson G.T, A.K Mallia and Smitjh P.K, Immobilized affinity ligand techniques. Academic press, 1992, 69-75) were taken in packed column and flow ELISA was carried out, in which MP at different concentrations and MP-HRP conjugates were used as described below. 2). 150 µl immobilized antibodies were packed in a glass capillary column and equilibrated with cold PBS (pH7.4, 50 mM) using 10 times of the bed volume. 50µl sample of this solution containing pesticide was passed through the column at 50µl/min flow rate. 3). PBS was continuously passed for 5 min. to wash of unbound pesticide. 4). MP-HRP conjugate 25µl, was passed through the column at 50µl/min flow rate. 5). The unbound conjugate was washed-out for 15 min using PBS containing 0.1% BSA. 6). Finally, the conjugate containing matrix was taken out from the column and used for the Chemiluminescence test as follows:
   To 50 µl conjugate containing matrix obtained from above, 25µl urea H₂O₂ (10mM in Milli Q) was added and kept at room temp for 2 min. Then 25µl of 0.1mM Luminol was added to 25µl of 0.5% K₃Fe (CN)₆, (prepared in Milli Q water) and mixed thoroughly. During this process light is generated which is immediately captured by CCD camera in dark room conditions. Intensity of light in the images were compared with control having no pesticide. It was found that there was less light produced in control whereas more light was produced in pesticide containing samples. It was also observed that more light was produced at higher pesticide concentrations whereas less light was observed with less pesticide containing samples.

The following example is given by way of illustration only and therefore should not be constructed to limit the scope of present invention.

### Example:

This example illustrates the present invention, which has been schematically shown in Fig 2. Referring to Fig. 2, 150µl immobilized antibodies were packed in a glass capillary column 6 and equilibrated with cold PBS (pH7.4, 50 mM) using 10 times of the bed volume. 50µl sample of solution containing pesticide 2 was passed through the column 6 at 50µl/min flow rate. PBS 1 was continuously passed for 5 min. to wash of unbound pesticide. MP-HRP conjugate 3 25µl, was passed through the column 6 at 50µl/min flow rate. The unbound conjugate was washed-out for 15 min using PBS 1 containing 0.1% BSA. All the above reagents are preferably passed into the column 6 through a peristaltic pump 5. Finally, the conjugate containing matrix was taken out from the column and used for the Chemiluminescence test conducted as follows:
To 50 µl conjugate containing matrix obtained from above, 25µl urea H₂O₂ (10mM in Milli Q) was added and kept at room temp for 2 min. Then 25µl of 0.1mM Luminol was added to 25µl of 0.5% K₃Fe (CN)₆, and mixed thoroughly as shown in 7. During this process, light is generated which is immediately captured by CCD camera 8 in dark room conditions. Intensity of light in the images were compared with control having no pesticide through a computer 9.

ICL intensity was monitored at different concentrations of the pesticide (MP) viz., 100ppb, 1ppb, 500ppt and 10 ppt along with the control, which did not have any pesticide. The clear observable CL intensity can be identified upto 10ppt. As can be seen from the bottom of Fig 2, at lower pesticide concentration CL difference between sample and control is not distinguishable. By this test kit device it was possible to detect presence and absence of the pesticide up to 10 ppt.

The simple photo detection kit for pesticide analysis of the present invention enables testing for pesticide in water, food and environment even by a common man.

## Claims

1. A method for rapid detection of pesticide at ppt level using immuno-chemiluminescence which comprises:
packing immobilized antibodies packed in a glass capillary column, passing a sample solution to be tested for pesticide content through said column so that a pesticide-antibody conjugate is formed, removing said pesticide-antibody conjugate from the column and adding thereto, urea H₂O₂ followed by Luminol and K₃Fe(CN)₆, mixing said solution thoroughly until light is generated, and capturing the light generated by a CCD camera, the intensity of the light produced being directly proportional to the amount of pesticide content in said sample.

2. A method as claimed in claim 1 wherein said antibodies comprise methyl parathion (MP) antibodies raised in chicken and purified from egg yolk (IgY- MP) and immobilized on sepharose matrix.

3. A method as claimed in claim 1 or 2 wherein said Urea H₂O₂ is employed in the range of 10-50µl.

4. A method as claimed in any preceding claim wherein said Luminol is employed in the range of 10-50µl.

5. A method as claimed in any preceding claim wherein said K3FeCN6 is employed in the range of 10-150µl.

6. A method as claimed in an preceding claim wherein after the addition of urea H₂O₂, said pesticide-antibody conjugate is maintained at room temperature for about two minutes.

7. A method as claimed in any preceding claim wherein said column is fed with phosphate buffer to wash unbound pesticide.

8. A kit for rapid detection of pesticide at ppt level using immuno-chemiluminescence which comprises a capillary column comprising packed immobilized antibodies, a source of sample solution to be tested for pesticide content, said source of sample solution being operatively connected to said column, a source of pesticide operatively connected to said column, a source of buffer operatively connected to said column, a source of urea H₂O₂ operatively connected to said column, a source of Luminol operatively connected to said column, and a source of K₃Fe(CN)₆ operatively connected to said column so that all the reagents are thoroughly mixed until light is generated, and capturing the light generated and a CCD camera to record the intensity of the light produced as a function of level of pesticides present in said sample.

9. A kit as claimed in claim 7 wherein a computer is connected to said CCD camera to compare the light produced by the test sample with a control sample.

## Patentansprüche

1. Ein Verfahren zur schnellen Detektion eines Pestizids im ppt Niveau unter Verwendung von Immunochemilumineszenz, das umfasst:
Packen immobilisierter Antikörper, die in einer Glaskapillarsäule gepackt sind, Durchleiten einer Probelösung, die auf einen Pestizidgehalt getestet werden soll, durch die Säule, so dass ein Pestizid-Antikörper-Konjugat gebildet wird, Entfernen des Pestitzid-Antikörper-Konjugats von der Säule und Hinzufügen von Harnstoff H₂O₂ gefolgt von Luminol und K₃Fe(CN)₆, gründliches Mischen der Lösung bis Licht gebildet wird und Auffangen des gebildeten Lichts durch eine CCD Kamera, wobei die Intensität des erzeugten Lichts direkt proportional zu der Menge des Pestizidgehalts in der Probe ist.

2. Ein Verfahren wie in Anspruch 1 beansprucht, wobei die Antikörper Methylparathion (MP) Antikörper umfassen, die in Hühnern gezüchtet und aus Eigelb (IgY-MP) gereinigt und auf Sepharosematrix immobilisiert wurden.

3. Ein Verfahren wie in Anspruch 1 oder 2 beansprucht, wobei das Harnstoff H₂O₂ im Bereich von 10 - 50 µl verwendet wird.

4. Ein Verfahren wie in einem der vorangegangenen Ansprüche beansprucht, wobei das Luminol im Bereich von 10 - 50 µl verwendet wird.

5. Ein Verfahren wie in einem der vorangegangenen Ansprüche beansprucht, wobei das K₃Fe(CN)₆ im Bereich von 10 - 50 µl verwendet wird.

6. Ein Verfahren wie in einem der vorangegangenen Ansprüche beansprucht, wobei nach der Zugabe des Harnstoff H₂O₂ das Pestizid-Antikörper-Konjugat für ungefähr zwei Minuten bei Raumtemperatur gehalten wird.

7. Ein Verfahren wie in einem der vorangegangenen Ansprüche beansprucht, wobei die Säule mit Phosphatpuffer gespeist wird, um ungebundenes Pestizid zu waschen.

8. Ein Kit zur schnellen Detektion eines Pestizids im ppt Niveau unter Verwendung von Immunochemilumineszenz, das eine Kapillarsäule, die gepackte, immobilisierter Antikörper umfasst, eine Quelle einer Probelösung, die auf einen Pestizidgehalt getestet werden soll, wobei die Quelle der Probelösung operativ mit der Säule verbunden ist, eine Quelle eines Pestizids, die operativ mit der Säule verbunden ist, eine Quelle eines Puffers, die operativ mit der Säule verbunden ist, eine Quelle von Harnstoff H₂O₂, die operativ mit der Säule verbunden ist, eine Quelle von Luminol, die operativ mit der Säule verbunden ist, und eine Quelle von K₃Fe(CN)₆, die operativ mit der Säule verbunden ist, so dass alle Reagenzien gründlich gemischt werden bis Licht erzeugt wird, das Auffangen des gebildeten Lichts und eine CCD Kamera umfasst, um die Intensität des erzeugten Lichts als eine Funktion des Niveaus an Pestiziden, die in der Probe vorliegen, aufzunehmen.

9. Ein Kit wie in Anspruch 7 beansprucht, wobei ein Computer mit der CCD Kamera verbunden ist, um das Licht, das durch die Testprobe erzeugt wurde, mit einer Kontrollprobe zu vergleichen.

## Revendications

1. Procédé de détection rapide de pesticide à un niveau ppt en utilisant l'immunochimioluminescence qui consiste à :
charger des anticorps immobilisés chargés dans une colonne capillaire en verre, faire passer une solution d'échantillon destinée à être testée en ce qui concerne sa teneur en pesticide à travers ladite colonne de sorte qu'un conjugué pesticide-anticorps soit formé, retirer ledit conjugué pesticide-anticorps de la colonne et y ajouter, de l'urée H₂O₂ suivi par du Luminol et K₃Fe(CN)₆, mélanger complètement ladite solution jusqu'à ce que de la lumière soit générée, et capturer la lumière générée par une caméra CCD, l'intensité de la lumière produite étant directement proportionnelle à la quantité de teneur en pesticide dans ledit échantillon.

2. Procédé selon la revendication 1, dans lequel lesdits anticorps comprennent des anticorps anti méthyle-parathion (MP) produits chez le poulet et purifiés à partir de jaune d'oeuf (IgY-MP) et immobilisés sur une matrice de sépharose.

3. Procédé selon la revendication 1 ou 2, dans lequel ladite urée H₂O₂ est employée dans le domaine de 10 à 50 µl.

4. Procédé selon l'une quelconque des revendications précédentes, dans lequel ledit Luminol est employé dans le domaine de 10 à 50 µl.

5. Procédé selon l'une quelconque des revendications précédentes, dans lequel ledit K₃FeCN₆ est employé dans le domaine de 10 à 150 µl.

6. Procédé selon l'une quelconque des revendications précédentes, dans lequel après l'addition de l'urée H₂O₂, ledit conjugué pesticide-anticorps est maintenu à température ambiante pendant environ deux minutes.

7. Procédé selon l'une quelconque des revendications précédentes, dans lequel ladite colonne est alimentée de tampon phosphate pour laver le pesticide non lié.

8. Kit de détection rapide de pesticide à un niveau ppt utilisant l'immunochimioluminescence qui comprend une colonne capillaire comprenant des anticorps immobilisés chargés, une source de solution d'échantillon destinée à être testée en ce qui concerne sa teneur en pesticide, ladite source de solution d'échantillon étant reliée de manière fonctionnelle à ladite colonne, une source de pesticide reliée de manière fonctionnelle à ladite colonne, une source de tampon reliée de manière fonctionnelle à ladite colonne, une source d'urée H₂O₂ reliée de manière fonctionnelle à ladite colonne, une source dé Luminol reliée de manière fonctionnelle à ladite colonne, et une source de K₃Fe(CN)₆ reliée de manière fonctionnelle à ladite colonne de sorte que tous les réactifs soient mélangés complètement jusqu'à ce que de la lumière soit générée, et la capture de la lumière générée et une caméra CCD pour enregistrer l'intensité de la lumière produite en fonction du niveau de pesticide présent dans ledit échantillon.

9. Kit selon la revendication 7, dans lequel un ordinateur est relié à ladite caméra CCD pour comparer la lumière produite par l'échantillon d'essai avec un échantillon témoin.
